# EUROPEAN PATENT SPECIFICATION

(11) **EP 1 423 360 B1**
(45) Date of publication and mention of the grant of the patent: **25.04.2007**
(21) Application number: 02758720.3
(22) Date of filing: 23.08.2002
(51) Int. Cl.: C07C 319/28, C07C 323/59

(54) **PROCESS FOR THE PREPARATION OF CILASTATIN**
VERFAHREN ZUR HERSTELLUNG VON CILASTATIN
PROCEDE DE PREPARATION DE CILASTATINE

(30) Priority: 24.08.2001 IN DE08792001
(43) Date of publication of application: 02.06.2004
(73) Proprietor: RANBAXY LABORATORIES, LTD., Gurgaon 122 001 HR (IN)
(72) Inventor: KUMAR, Yatendra, 122001 Gurgaon, Haryana (IN); TYAGI, Om, Dutt, 122001 Gurgaon, Haryana (IN); SRIVASTAVA, Tushar, Kumar, c/o Dr. Narendra Kumar, 226020 Aliganj, Lucknow (IN); PANDEY, Anand, 230128 Pratap Garh, Uttar Pradesh (IN)
(74) Representative: Cronin, Brian Harold John
(86) International application number: PCT/IB2002/003399
(87) International publication number: WO 2003/018544

(56) References cited:
- US-A- 5 147 868

## Description

### Field of the Invention

The present invention relates to an efficient and industrially advantageous process for the preparation of pure cilastatin.

### Background of the Invention

Cilastatin possesses the ability to prevent nephrotoxicity associated with the use of β-lactam antibiotics such as imipenem. Chemically, cilastatin is [R-{R*, S-(Z)]]-7-[(2-amino-2-carboxyethyl)thio]-2-[[2,2-dimethylcyclopropyl)carbonyl]amino-2-heptenoic acid and has the structural Formula I.

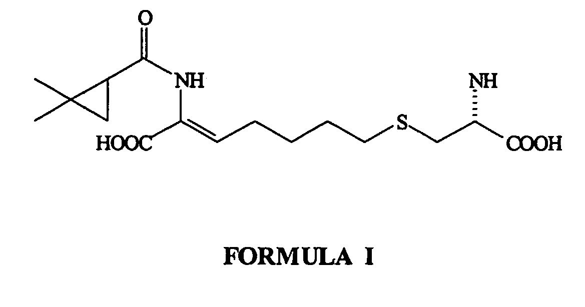

It is a renal dehydropeptidase inhibitor and is co-administered as the sodium salt with imipenem in order to prevent its renal metabolism. lmipenem/cilastatin combination is used as a potent broad spectrum antibacterial ageht.

Cilastatin was first disclosed in U.S. Patent No. 5,147,868 and was obtained in a multi-step synthesis involving condensing cysteine hydrochloride with heptenoic acid of Formula II, 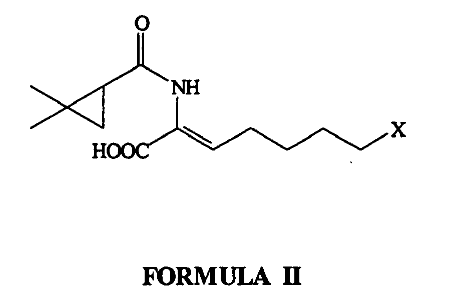 wherein X is chloro or bromo, in the presence of sodium hydroxide in aqueous medium. Cilastatin so obtained contains the corresponding undesired E-isomer in amounts ranging from about 6 to 10% as determined by HPLC. A process for isomerising the E-isomer to cilastatin by heating the mixture at pH 3 is also disclosed. However, we have observed that the isomerization process results in the formation of impurities in the range of 5-8% due to the degradation of cilastatin which renders the product produced by this process unsuitable for human consumption.

U.S. Patent No. 5,147,868 also teaches a method for isolating cilastatin from the reaction mixture involving two purifications viz. chromatography using a cation exchange resin followed by solvent purification using ethanol and diethyl ether. The ion exchange chromatography removes inorganic salts such as sodium chloride which is otherwise difficult to remove as cilastatin itself is also water soluble.

However, our attempts to isolate pure cilastatin by following the process exemplified in said patent were unsuccessful. We could obtain cilastatin in the form of its ammonium salt on eluting the cation exchange resin with ammonia solution and not as the free acid. Obtaining the free acid by using an acid such as hydrochloric acid entailed formation of inorganic ammonium salts such as ammonium chloride thus defeating the very purpose of using a cation exchange resin.

J. Med. Chem. 1987; 30: 1083 discloses a process for the preparation of cilastatin involving the condensation of cysteine with the halo-heptenoic acid of Formula II in sodium metal/liquid ammonia and the resultant mixture is isomerized to obtain cilastatin using methyl iodide in methanol. Cilastatin is isolated by using a cation exchange resin followed by the treatment with an anion exchange resin to remove the inorganic salts.

However, this process is not suitable on an industrial scale as it involves the use of sodium metal/liquid ammonia which are very hazardous and also uses methyl iodide for isomerization, which is expensive and requires special storage conditions. More over, loading the ion exchange column requires repeated circulation of cilastatin solution. Also, ion exchange column operates on the principle of ionic bonding / acid base reaction. Such reaction being exothermic causes considerable degradation of cilastatin. The use of two stage ion exchange chromatography is cumbersome, tedious and not practicable on an industrial scale.

In light of the above drawbacks in the prior art processes, there is a need for the development of a simple, convenient and efficient process for the preparation of pure cilastatin which is convenient to operate on an industrial scale.

### Summary of the Invention

The present invention provides a process for the purification of cilastatin using a non-ionic adsorbent resin. The process requires a single purification using chromatographic technique to obtain the pure product. Loading of the non-ionic adsorbent resin with crude cilastatin is achieved by passing the solution only once through the resin. Since no acid base reaction takes place, no degradation of the product is observed. The present invention thus fulfills the need for a process which is convenient to operate on an industrial scale.

Accordingly, the present invention provides a process for the purification of cilastatin which comprises contacting a solution of crude cilastatin with a non-ionic adsorbent resin and recovering pure cilastatin from a solution thereof.

In the meaning of the present invention, the term "crude cilastatin" comprises cilastatin containing impurities which may be inorganic salts such as sodium chloride, sodium bromide and the like, or organic impurities which may have formed due to the degradation of cilastatin, or the side products formed during the synthesis, or unreacted intermediates of the multi-step synthesis for the preparation of cilastatin.

The solution of crude cilastatin may be obtained by dissolving the crude cilastatin in a suitable solvent or may be obtained directly from the reaction mixture for the preparation of cilastatin containing already dissolved crude cilastatin. The term "suitable solvents" as used herein includes water, organic solvents, and mixtures thereof. The organic solvents include methanol, ethanol, acetonitrile, acetone, and the like. The crude cilastain may be prepared by any of the methods reported in prior art.

Any of the non-ionic adsorbent resins which are commercially available and on the surface of which cilastatin is adsorbed, may be used. In particular, non-ionic macroporous water insoluble polymers such as polyacrylates or copolymers of styrene and polyvinyl benzene may be used. Preferred adsorbent resin is a copolymer of styrene cross linked with divinylbenzene.

In the meaning of the present invention, the term "pure cilastatin" refers to cilastatin having a purity of 98% or more by HPLC.

A typical process for the purification of cilastatin comprises loading a solution of crude cilastatin on a column of non-ionic adsorbent resin, washing it with deionized water till no halide ions can be detected. The resin is then eluted with organic or aqueous organic solvent and pure cilastatin is isolated from the eluate by common methods known in the art such as concentration, precipitation and recrystallization as required. However, alternative method of purification such as slurrying with the adsorbent resin may also be used.

According to another aspect of the present invention, it provides a process for the isomerization of E-isomer to cilastatin. The process comprises heating a solution of cilastatin containing the corresponding undesired E-isomer at a pH of about 0.5 to 1.5. We have observed that cilastatin obtained using this process greatly reduces the formation of degradation products.

The solution of cilastatin containing the corresponding E-isomer is preferably obtained directly from the reaction mixture for the preparation of cilastatin. Cilastatin may be prepared by any of the multi-step processes described in prior art.

The isomerization is preferably performed at 85-95°C. The pH is adjusted to 0.5 to 1.5, more preferably to 0.5 to 1 and most preferably to about 0.5. Any acid may be used for adjusting the pH of the solution. Preferably, hydrochloric acid is used.

In a preferred embodiment of the present invention, the two aspects of the invention are combined i.e. the isomerization process is followed by the purification process to obtain pure cilastatin.

### Detailed Description of the Invention

In the following section preferred embodiments are described by way of examples to illustrate the process of the invention. However, these are not intended in any way to limit the scope of the present invention.

### EXAMPLE 1

### Preparation of cilastatin

Cysteine hydrochloride monohydrate (166.3g) was dissolved in water (1.2L). To this solution, aqueous sodium hydroxide (113.7 g in 400ml water) and sodium salt of 7-chloro-2-[[(1S)-2,2-dimethylcyclopropane]carboxamido]-2-heptenoic acid (200g) were added. The reaction mixture was stirred at room temperature. The corresponding E isomer (5% by HPLC) was isomerized to cilastatin by heating the reaction mixture at 85-90°C for 30 minutes after adjusting the pH to 0.5 with concentrated hydrochloric acid.

### Purification of cilastatin

The reaction mixture obtained above was loaded on a column packed with diaion HP - 20 resin as the adsorbent. The column was washed with water to remove sodium chloride and then the product was eluted with aqueous methanol. The column fractions containing the pure product were pooled and concentrated to obtain pure cilastatin (160g; Purity : 99 % by HPLC).

### EXAMPLE 2

Ethyl-7-chloro-2-oxo-heptanoate (25g), (S)-2,2-dimethylcyclopropane carboxamide (13.68g) and p-toluene sulphonic acid (0.125g) were refluxed in toluene using dean - stark trap for the azeotropic removal of water from the reaction mixture. After the condensation was complete, the reaction mixture was washed with dilute hydrochloric acid and aqueous sodium bisulfite to remove the unreacted (S)-2,2-dimethylcyclopropane carboxamide and ethyl-7-chloro-2-oxo-heptanoate, respectively. The organic solution was then concentrated to recover toluene under reduced pressure. The resultant oily ethyl ester of 7-chloro-2-[[(1S)-2,2-dimethylcyclopropane]carboxamide]-2-heptenoic acid was hydrolyzed with aqueous sodium hydroxide in the presence of denatured spirit at room temperature. After hydrolysis, the reaction mixture was concentrated to half of its volume under reduced pressure and washed with toluene. Cysteine hydrochloride monohydrate (29.7g) and aqueous sodium hydroxide solution were added to the above aqueous layer. The reaction mixture was stirred at room temperature till the complete conversion of 7-chloro-2-[[(1S)-2,2-dimethylcyclopropane]carboxamido]-2-heptenoic acid to the product was achieved. The corresponding E isomer was isomerized to cilastatin by heating the reaction mixture at 85-90°C after adjusting the pH to 0.5 with concentrated hydrochloric acid.

### Purification of cilastatin

The reaction mixture obtained above was loaded on a column packed with diaion HP 20 resin as the adsorbent. The column was washed with water to remove sodium chloride and then the product was eluted with aqueous acetonitrile. The column fractions containing the pure product were pooled and concentrated to obtain pure cilastatin (16.3g; Purity :99.2% by HPLC).

## Claims

1. A process for the purification of cilastatin which comprises contacting a solution of crude cilastatin with a non-ionic adsorbent resin and recovering pure cilastatin from a solution thereof.

2. The process of claim 1 wherein the solution of crude cilastatin is obtained directly from the reaction mixture.

3. The process of claim 1 wherein the solution of crude cilastatin is obtained by dissolving crude cilastatin in a suitable solvent.

4. The process of claim 3 wherein the suitable solvent is selected from water, an organic solvent, and mixture(s) thereof.

5. The process of claim 4 wherein the organic solvent is selected from the group consisting of methanol, ethanol, acetonitrile and acetone.

6. The process of claim 1 wherein the non-ionic adsorbent resin comprises a non-ionic macroporous water insoluble polymer.

7. The process of claim 6 wherein the polymer is selected from the group consisting of polyacrylates or copolymers of styrene and polyvinyl benzene.

8. The process of clam 7 wherein the polymer is a copolymer of styrene cross linked with divinylbenzene.

9. A process for the preparation of cilastatin comprising heating a solution of cilastatin containing the corresponding E-isomer at a pH of about 0.5 to 1.5.

10. The process of claim 9 wherein the solution is heated to 85-95°C.

11. The process of claim 9 wherein the solution is heated to 85-95°C at a pH of about 0.5.

12. A process for the preparation of pure cilastatin comprising:
(i) heating a solution of cilastatin containing the corresponding E-isomer at a pH of about 0.5 to 1.5, and
(ii) contacting the solution obtained with a non-ionic adsorbent resin and recovering pure cilastatin from a solution thereof.

## Patentansprüche

1. Verfahren zur Reinigung von Cilastatin, umfassend das Inkontaktbringen einer Lösung rohen Cilastatins mit einem nichtionischen adsorbierenden Kunstharz und Zurückgewinnen des reinen Cilastatins aus einer Lösung davon.

2. Verfahren nach Anspruch 1, wobei die Lösung des rohen Cilastatins unmittelbar aus dem Reaktionsgemisch gewonnen wird.

3. Verfahren nach Anspruch 1, wobei die Lösung des rohen Cilastatins durch Lösen rohen Cilastatins in einem geeigneten Lösungsmittel gewonnen wird.

4. Verfahren nach Anspruch 3, wobei das geeignete Lösungsmittel aus Wasser, einem organischen Lösungsmittel und (einer) Mischung(en) davon ausgewählt ist.

5. Verfahren nach Anspruch 4, wobei das organische Lösungsmittel aus der aus Methanol, Ethanol, Acetonitril und Aceton bestehenden Gruppe ausgewählt ist.

6. Verfahren nach Anspruch 1, wobei das nichtionische adsorbierende Kunstharz ein nichtionisches makroporöses wasserunlösliches Polymer umfasst.

7. Verfahren nach Anspruch 6, wobei das Polymer aus der aus Polyacrylaten oder Copolymeren aus Styrol und Polyvinylbenzol bestehenden Gruppe ausgewählt ist.

8. Verfahren nach Anspruch 7, wobei das Polymer ein mit Divinylbenzol vernetztes Styrolpolymer ist.

9. Verfahren zur Herstellung von Cilastatin, umfassend das Erhitzen einer Lösung von Cilastatin, welche das entsprechende E-Isomer enthält, bei einem pH von etwa 0,5 bis 1, 5.

10. Verfahren nach Anspruch 9, wobei die Lösung auf 85 - 95 °C erhitzt wird.

11. Verfahren nach Anspruch 9, wobei die Lösung bei einem pH von etwa 0,5 auf 85 - 95 °C erhitzt wird.

12. Verfahren zur Herstellung von reinem Cilastatin, umfassend
(i) Erhitzen der das entsprechende E-Isomer enthaltenden Cilastatinlösung bei einem pH von etwa 0,5 bis 1,5 und
(ii) Inkontaktbringen der Lösung mit einem nichtionischen adsorbierenden Kunstharz und Zurückgewinnen des reinen Cilastatins aus einer Lösung davon.

## Revendications

1. Procédé de purification de cilastatine qui consiste à mettre en contact une solution de cilastatine brute avec une résine adsorbante non ionique et à récupérer de la cilastatine pure à partir d'une solution de celle-ci.

2. Procédé de la revendication 1, dans lequel la solution de cilastatine brute est obtenue directement à partir du mélange réactionnel.

3. Procédé de la revendication 1, dans lequel la solution de cilastatine brute est obtenue en dissolvant la cilastatine brute dans un solvant approprié.

4. Procédé de la revendication 3, dans lequel le solvant approprié est choisi parmi l'eau, un solvant organique et un ou des mélanges de ceux-ci.

5. Procédé de la revendication 4, dans lequel le solvant organique est choisi parmi le groupe constitué de méthanol, éthanol, acétonitrile et acétone.

6. Procédé de la revendication 1, dans lequel la résine adsorbante non ionique comprend un polymère insoluble dans l'eau macroporeux non ionique.

7. Procédé de la revendication 6, dans lequel le polymère est choisi parmi le groupe constitué de polyacrylates ou de copolymères de styrène et de polyvinylbenzène.

8. Procédé de la revendication 7, dans lequel le polymère est un copolymère de styrène réticulé avec du divinylbenzène.

9. Procédé de préparation de cilastatine, consistant à chauffer une solution de cilastatine contenant l'isomère E correspondant à un pH d'environ 0,5 à 1,5.

10. Procédé de la revendication 9, dans lequel la solution est chauffée à 85-95°C.

11. Procédé de la revendication 9, dans lequel la solution est chauffée à 85-95°C à un pH d'environ 0,5.

12. Procédé de préparation de cilastatine pure consistant à :
(i) chauffer une solution de cilastatine contenant l'isomère E correspondant à un pH d'environ 0,5 à 1,5, et
(ii) mettre en contact la solution obtenue avec une résine adsorbante non ionique et récupérer la cilastatine pure à partir d'une solution de celle-ci.
